# EUROPEAN PATENT SPECIFICATION

(11) **EP 1 172 363 B1**
(45) Date of publication and mention of the grant of the patent: **25.05.2005**
(21) Application number: 01116014.0
(22) Date of filing: 02.07.2001
(51) Int. Cl.: C07D 311/72

(54) **Acylation process**
Acylierungsverfahren
Procédé d'acylation

(30) Priority: 10.07.2000 EP 00114768
(43) Date of publication of application: 16.01.2002
(73) Proprietor: DSM IP Assets B.V., 6411 TE Heerlen (NL)
(72) Inventor: Bonrath, Werner, 79115 Freiburg (DE); Cirillo, Fabio, 8406 Winterthur (CH)
(74) Representative: Müller, Ingrid, Dr.

(56) References cited:
- WO-A-97/28151
- FR-A- 2 666 581
- US-A- 2 723 278
- N. COHEN ET AL.: HELVETICA CHIMICA ACTA, vol. 64, no. 4, 1981, pages 1158-73, XP001030848
- GIALIH LIN ET AL.: TETRAHEDRON LETTERS, vol. 39, no. 24, 1998, pages 4333-6, XP001030881
- A. MORCUENDE ET AL.: JOURNAL OF ORGANIC CHEMISTRY, vol. 61, no. 16, 1996, pages 5264-70, XP002181494
- B. HERRAD N ET AL.: SYNLETT, no. 5, 1995, pages 455-8, XP001030883

## Description

The present invention is concerned with a novel process for the preparation of tocol and tocopherol acylates. Tocol is the compound 2-methyl-2(4,8,12-trimethyl-tridecyl)-chroman-6-ol. The term tocopherol as used herein refers to all the compounds derived from the basic structure of tocol and having vitamin E character, viz., the tocopherols having a saturated (tocol) side chain, such as α-, β-, γ-, δ-, ζ₂- and η-tocopherol, and the tocotrienols having three double bonds in the side chain such as ε- and ζ₁- tocopherol. Of the various tocopherols, (all-rac)-α-tocopherol (generally referred to as vitamin E) is of primary interest.

The present invention is preferably concerned with a novel process for the preparation of tocopherol acylates, more particularly tocopherol acetates. The main commercial form of vitamin E being (all-rac)-α-tocopherol acetate, the invention, in a preferred aspect, is concerned with a process for the preparation of (all-rac)-α-tocopherol acetate. However, other tocopherols such as those mentioned above can be readily acylated by the process of the present invention. Further, by the process of the instant invention, the tocopherols can be acylated in the form of their racemates or individual enantiomers.

The synthesis of (all-rac)-α-tocopherol acetate starting from acetic anhydride and (all-rac)-α-tocopherol without a catalyst in an excess of acetic anhydride has been reported by Surmatis et al., USP 2,723,278. Under reflux conditions for three hours, the product was formed. The yield was not given. This reaction can also be carried out in the presence of pyridine as catalyst at room temperature to yield, after a reaction time of three days, 96 % (all-rac)-α-tocopherol acetate, see Cohen et al., Helv. Chim. Acta 1981, 64, 1158.

In accordance with the present invention it has been found that the acylation of tocol and tocopherols can advantageously be effected under irradiation with microwaves. As compared to prior art processes, the process of the present invention requires shorter reaction time, gives better yield and facilitates work-up of the reaction mixture. In particular, the process of this invention does not necessarily require external heating of the reaction mixture, thus providing uniform reaction conditions throughout the entire reaction mixture.

Accordingly, the present invention is concerned with a novel process for the preparation of tocol or tocopherol acylates which process comprises reacting tocol or a tocopherol with an acylating agent, in the presence of an organic base or an organic or inorganic acid as the catalyst or in the absence of a catalyst, and under irradiation with microwaves.

The term "microwave" as used herein refers to the region of the electromagnetic spectrum having frequencies of 30 GHz to 300 MHz thus corresponding to wavelengths of 1 cm to 1 m. In order not to interfere with wavelengths for Radar (1 cm - 25 cm), household or industrial microwave heaters are required to operate at either 12.2 cm (2.45 GHz) or 33.3 cm (918 MHz). Thus, in a preferred embodiment of the invention, the term microwaves refers particularly to such wavelenghts. In the process of this invention, conventional microwave equipment can be used. Microwave equipment suitable in the process of this invention is supplied, e.g., by the firms MLS, Leutkirch, Germany (Lavis Multiquant 1000); or MILESTONE Inc., Monroe,CT 06468, USA (Ethos reactors). Conveniently, the irradiation in the process of this invention is carried out applying a power of irradiation of from about 600 to 1200 W, more preferably from about 800 to about 1000 W.

The acylation can be carried out using any acylating agent conventionally used for the acylation of a phenolic hydroxy group as is present in tocol and tocopherols, e.g., acyl anhydrides or halogenides. The acyl groups in such acylating agent may be derived from aliphatic carboxylic acids, e.g., from alkanoic acids, in particular C1-7 -alkanoic acids as acetic acid, propionic acid, butyric acid or pivalic acid, or higher alkanoic acids such as palmitic acid; or from aromatic carboxylic acids, e.g., benzoic acid; or araliphatic acids, e.g. phenylacetic acid.

The acylation can be carried out in the presence or in the absence of a catalyst such as an organic base, e.g. pyridine or dimethylamino pyridine, or an organic or inorganic acid, e.g., sulfuric acid or p-toluenesulfonic acid. Advantageously, if a catalyst is used, a volatile catalyst is elected. In a preferred aspect of this invention, the acylation is carried out in the absence of a catalyst. The acylating agent is suitably used in excess, i.e. in an excess of about 100 % over the stoichiometrically required amount. Suitably, the reaction is carried out in an inert atmosphere. The desired tocol or tocopherol acylate can be isolated from the reaction product by conventional means, e.g., by heating the reaction mixture under reduced pressure to remove excess acylating agent and catalyst, if any, and other unwanted products. While the process of the present invention is preferably concerned with the acylation of (all-rac)-tocopherols, particularly (all-rac)-α-tocopherol, it is to be understood that it can be used to acylate optically pure enantiomers, such as (d-)-α-tocopherol.

The invention is further illustrated by the Examples which follow.

### Example 1

(all-rac)-α-Tocopherol (52.63g, 95 % pure, corresponding to 116 mmol) and acetic anhydride (22.69 ml; 242 mmol) were placed in a flask. Pyridine (0.31ml, 3.9 mmol) was added at once to the reaction mixture. The reaction mixture was stirred and irradiated with 800 W or 1000 W microwaves using a LAVIS Multiquant reactor under inert gas atmosphere (Ar). The reaction product obtained was purified by heating to 70 °C under 25mbar. The residue was analyzed by GC (XTI-5, 30m x 0.32 mm, Film 0.25 mm, fused silica; 150°C (0 min) ® 5°C./min ® 335°C. (8min), He 2.0 ml/min) against an internal standard (1.0 g octacosan in 100 ml n-heptane). The conversion of tocopherol to its acetate after 5, 10, 20 and 30 minutes is shown in Tables 1 and 2 below. Deviations in the sum of the percentages from 100 are due to analytical errors.

**Table 1:**

| Conversion of tocopherol to tocopherol acetate; catalyst: pyridine; 800 W | | | | |
|---|---|---|---|---|
| min | 5 | 10 | 20 | 30 |
| tocopherol acetate [%] | 43 | 94 | 99 | 99 |
| tocopherol [%] | 48 | 0 | 0 | 0 |

**Table 2:**

| Conversion of tocopherol to tocopherol acetate; catalyst: pyridine; 1000 W | | | | |
|---|---|---|---|---|
| min | 5 | 10 | 20 | 30 |
| tocopherol acetate [%] | 98 | 98 | 100 | 100 |
| tocopherol [%] | 1 | 0 | 0 | 0 |

When carrying out the above reaction under conventional conditions (no irradiation, heating to 100 °C,) 91 % of tocopherol acetate and 0 % of tocopherol were found in the reaction product after 30 minutes reaction time.

### Example 2

The acetylation of tocopherol was carried out as in Example 1 but in the absence of catalyst (pyridine). The conversion of tocopherol to its acetate after 5, 10, 20 and 30 minutes is shown in Tables 3 and 4 below. Deviations in the sum of the percentages from 100 are due to analytical errors.

**Table 3:**

| Conversion of tocopherol to tocopherol acetate; no catalyst; 800 W | | | | |
|---|---|---|---|---|
| min | 5 | 10 | 20 | 30 |
| tocopherol acetate [%] | 62 | 76 | 97 | 99 |
| tocopherol [%] | 37 | 24 | 3 | 1 |

**Table 4:**

| Conversion of tocopherol to tocopherol acetate; no catalyst; 1000W | | | | |
|---|---|---|---|---|
| min | 5 | 10 | 20 | 30 |
| tocopherol acetate [%] | 73 | 86 | 98 | 99 |
| tocopherol [%] | 27 | 14 | 2 | 2 |

When carrying out the above reaction under conventional conditions (no irradiation, no catalyst, reflux temperature, reaction time 3,5 hours) the conversion of tocopherol to its acetate proceeded as shown in Table 5 below (deviations in the sum of the percentages from 100 being due to analytical errors)

**Table 5:**

| Conventional conversion of tocopherol to tocopherol acetate (no catalyst) | | | | |
|---|---|---|---|---|
| min | 30 | 60 | 90 | 120 |
| tocopherol acetate [%] | 69 | 93 | 94 | 95 |
| tocopherol [%] | 30 | 9 | 6 | 5 |

## Claims

1. Process for the preparation of tocol and tocopherol acylates which comprises reacting tocol or a tocopherol with an acylating agent in the presence of an organic base or an organic or inorganic acid as the catalyst or in the absence of a catalyst, and under irradiation with microwaves.

2. A process as in claim 1, wherein a tocopherol is acylated.

3. A process as in claim 2 wherein the tocopherol is (all-rac)-α-tocopherol.

4. A process as in any one of claim 1 to 3, wherein the acylating agent is an acetylating agent.

5. A process as in any one of claims 1 to 4, wherein the acetylating agent is acetic anhydride.

6. A process as in any one of claims 1 to 5 wherein the process is carried out in the absence of a catalyst.

7. A process as in any one of claims 1 to 6 wherein a microwave source of about 600 to about 1200 W is used.

8. A process as in claim 7 wherein a microwave source of about 800 to about 1000 W is used.

## Patentansprüche

1. Verfahren zur Herstellung von Tocol- und Tocopherolacylaten, umfassend die Umsetzung von Tocol oder eines Tocopherols mit einem Acylierungsmittel in der Gegenwart einer organischen Base oder einer organischen oder anorganischen Säure als den Katalysator oder in Abwesenheit eines Katalysators und unter Bestrahlung mit Mikrowellen.

2. Verfahren nach Anspruch 1, wobei ein Tocopherol acyliert wird.

3. Verfahren nach Anspruch 2, wobei das Tocopherol (all-rac)-α-Tocopherol ist.

4. Verfahren nach einem der Ansprüche 1 bis 3, wobei das Acylierungsmittel ein Acetylierungsmittel ist.

5. Verfahren nach einem der Ansprüche 1 bis 4, wobei das Acerylierungsmittel Essigsäureanhydrid ist.

6. Verfahren nach einem der Ansprüche 1 bis 5, wobei das Verfahren in Abwesenheit eines Katalysators durchgeführt wird.

7. Verfahren nach einem der Ansprüche 1 bis 6, wobei eine Mikrowellenquelle von etwa 600 bis etwa 1200 W verwendet wird.

8. Verfahren nach Anspruch 7, wobei eine Mikrowellenquelle von etwa 800 bis etwa 1000 W verwendet wird.

## Revendications

1. Procédé pour la préparation d'acylates de tocol et de tocophérol qui comprend la réaction de tocol ou de tocophérol avec un agent acylant en présence d'une base organique ou d'un acide organique ou inorganique comme catalyseur ou en l'absence de catalyseur, et sous irradiation avec des micro-ondes.

2. Procédé selon la revendication 1, dans lequel on acyle un tocophérol.

3. Procédé selon la revendication 2, dans lequel le tocophérol est le (all-rac)-α-tocophérol.

4. Procédé selon l'une quelconque des revendications 1 à 3, dans lequel l'agent acylant est un agent acétylant.

5. Procédé selon l'une quelconque des revendications 1 à 4, dans lequel l'agent acétylant est l'anhydride acétique.

6. Procédé selon l'une quelconque des revendications 1 à 5, dans lequel on réalise le procédé en l'absence de catalyseur.

7. Procédé selon l'une quelconque des revendications 1 à 6, dans lequel on utilise une source de micro-ondes d'environ 600 à environ 1200 W.

8. Procédé selon la revendication 7, dans lequel on utilise une source de micro-ondes d'environ 800 à environ 1000 W.
